# EUROPÄISCHE PATENTSCHRIFT

(11) **EP 0 508 943 B1**
(45) Veröffentlichungstag und Bekanntmachung des Hinweises auf die Patenterteilung: **14.12.1994**
(21) Anmeldenummer: 92810160.9
(22) Anmeldetag: 03.03.1992
(51) Int. Cl.: C07C 67/287, C07C 69/63

(54) **Verfahren zur Herstellung von halogenierten Carbonsäureestern**
Process for the preparation of halogenated esters of carboxylic acids
Procédé de préparation d'esters halogénés d'acides carboxyliques

(30) Priorität: 11.03.1991 CH 716/91
(43) Veröffentlichungstag der Anmeldung: 14.10.1992
(73) Patentinhaber: Säurefabrik Schweizerhall, CH-4133 Schweizerhalle (CH)
(72) Erfinder: Gallegra, Pasquale, Dr., CH-4132 Muttenz (CH); Degischer, Gerhard, Dr., CH-4414 Füllinsdorf (CH)
(74) Vertreter: Schluep, Hans-Peter

(56) Entgegenhaltungen:
- FR-A- 2 269 508
- SU-A- 1 313 850

## Beschreibung

Die Erfindung betrifft ein neuartiges Verfahren zur Herstellung von halogenierten Carbonsäureestern der Formel I 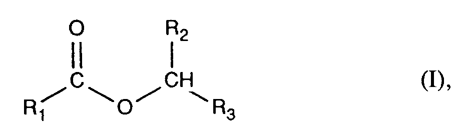
worin R₁ Alkyl, Alkenyl, Alkinyl, Cycloalkyl, Aralkyl oder Aryl bedeutet, R₂ für Wasserstoff, Alkyl oder Cycloalkyl steht und R₃ Halogen der Ordnungszahl 9 bis und mit 53 darstellt, dadurch gekennzeichnet, dass man ein Acylal der Formel II 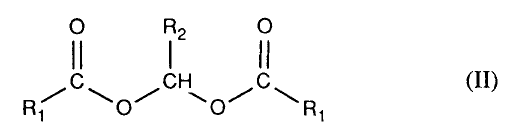
mit einem Halogenwasserstoff der Formel

H-R₃ (III)

umsetzt.

Alkyl bedeutet vorzugsweise Niederalkyl, wie geradkettiges oder verzweigtes C₁-C₇-Alkyl, wie Methyl, Ethyl, Propyl, Isopropyl, Butyl, Isobutyl, sec.-Butyl, tert.-Butyl, Pentyl, Isopentyl, sec.-Pentyl, Neopentyl oder eine Hexyl- oder Heptylgruppe, kann aber auch eine C₈-C₁₄-, wie Octyl-, Nonyl- oder Decylgruppe, sein. Als R₁ sind Methyl, Ethyl sowie sekundär-lineare und verzweigte C₃-C₇-Alkylgruppen, wie Isopropyl, But-2-yl, Pent-3-yl, tert.-Butyl oder 2-Methylbut-2-yl, und als R₂ lineare C₁-C₄-Alkylgruppen, wie Methyl, Ethyl, Propyl oder Butyl, bevorzugt.

Alkenyl bedeutet vorzugsweise geradkettiges oder verzweigtes C₂-C₇-Alkenyl, wie Ethenyl, Propenyl, z.B. Allyl, Isopropenyl, Methallyl (Crotyl), Butenyl oder 2-Methylprop-2-enyl.

Alkinyl ist beispielsweise C₃-C₇-Alkinyl, wie Propargyl.

Cycloalkyl ist beispielsweise 3- bis 8-, wie 5- bis 7-gliedriges Cycloalkyl, z.B. Cyclopropyl, Cyclobutyl, Cyclopentyl, Cyclohexyl oder Cycloheptyl.

Aryl ist beispielsweise Phenyl oder Naphthyl; Aralkyl ist beispielsweise Mono-, Di- oder Triphenyl-C₁-C₄-alkyl, wie Benzyl, 1-Phenylethyl, Diphenylmethyl oder Triphenylmethyl. Phenyl und Naphthyl sowie der Phenylteil von Mono-, Di- oder Triphenyl-C₁-C₄-alkyl können unsubstituiert oder durch übliche Substituenten, wie Niederalkyl, z.B. Methyl, Niederalkoxy, z.B. Methoxy, Halogen, z.B. Chlor oder Brom, Trifluormethyl und/oder Nitro substituiert, wie mono-, di- oder trisubstituiert sein, sind jedoch vorzugsweise unsubstituiert.

Halogen der Ordnungszahl 9 bis und mit 53 ist beispielsweise Chlor oder Brom, kann in zweiter Linie aber auch Jod sein.

Sofern nichts anderes angegeben ist, bedeutet der bei der Definition von Resten wie Niederalkyl und Niederalkoxy verwendete Ausdruck "Nieder", dass die betreffenden Reste bis und mit 7, vorzugsweise bis und mit 4 Kohlenstoffatome enthalten.

Die Verbindungen der Formel I sind wertvolle Zwischenprodukte in der organischen Synthese, insbesondere für die Herstellung von Arzneimittelwirkstoffen. Sie reagieren mit Aminen, Alkoholen und Carbonsäuren unter Einführung der Gruppe der Formel 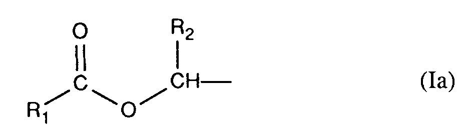
zu den entsprechenden substituierten Aminen, Ethern und Estern. Die Verbindungen der Formel I sind insbesondere geeignet für die Herstellung entsprechender Acyloxyalkylester von β-Lactamantibiotika, wie Penicillinen, pielsweise von 6-[D-(-)-α-Aminophenylacetamido]-penicillansäure(pivaloyloxy)methylester-hydrochlorid (Pivampicillin), und von Cephalosporinen, beispielsweise von 7-[2-Amino-4-thiazolyl)-(methoxyiminoacetylamino)-3-methyl-8-oxo]- 5-thia-1-azabicyclo[4.2.0]octencarbonsäure(pivaloyloxy)methylester (Cefetamet pivoxil) und dergleichen.

Die Erfindung betrifft in erster Linie die Herstellung von Verbindungen der Formel I, worin R₁ C₁-C₁₄-Alkyl, C₂-C₇-Alkenyl, C₃-C₇-Alkinyl, 3- bis 8-gliedriges Cycloalkyl, unsubstituiertes oder durch C₁-C₄-Alkyl, C₁-C₄-Alkoxy, Halogen, Trifluormethyl und/oder Nitro substituiertes Phenyl oder Naphthyl oder unsubstituiertes oder im Phenyl durch C₁-C₄-Alkyl, C₁-C₄-Alkoxy, Halogen, Trifluormethyl und/oder Nitro substituiertes Mono-, Di- oder Triphenyl-C₁-C₄-alkyl bedeutet, R₂ für Wasserstoff, C₁-C₇-Alkyl oder 3- bis 8-gliedriges Cycloalkyl steht und R₃ Halogen der Ordnungszahl 9 bis und mit 53 bedeutet.

Die Erfindung betrifft insbesondere die Herstellung von Verbindungen der Formel I, worin R₁ C₁-C₇-Alkyl, wie Methyl oder tert.-Butyl, bedeutet, R₂ für Wasserstoff oder C₁-C₄-Alkyl steht und R₃ Chlor oder Brom darstellt.

Die Erfindung betrifft vorzugsweise die Herstellung von Verbindungen der Formel I, worin R₁ Methyl, Ethyl oder sekundär-lineares oder verzweigtes C₃-C₇-Alkyl, wie Isopropyl oder tert.-Butyl, bedeutet, R₂ für Wasserstoff oder in zweiter Linie für lineares C₁-C₄-Alkyl, wie Methyl oder Ethyl, steht und R₃ Chlor oder in zweiter Linie Brom darstellt.

Die Erfindung betrifft in allererster Linie die Herstellung von Verbindungen der Formel I, worin R₁ Methyl, Ethyl oder sekundär-lineares oder verzweigtes C₃-C₇-Alkyl, wie Isopropyl oder tert.-Butyl, bedeutet, R₂ für Wasserstoff steht und R₃ Chlor darstellt.

Die Erfindung betrifft namentlich die Herstellung der in den Beispielen genannten Verbindungen der Formel I, insbesondere von Chlormethylpivalat (R₁ = tert.-Butyl, R₂ = Wasserstoff).

Das übliche Verfahren zur Herstellung von Verbindungen der Formel I ist dadurch gekennzeichnet, dass man ein Säurehalogenid der Formel IV 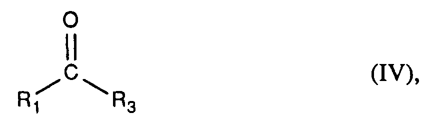
worin R₁ Methyl oder tert.-Butyl und R₃ Chlor oder Brom bedeutet, in Gegenwart eines entsprechenden Zinkhalogenides mit Paraformaldehyd kondensiert oder ein annähernd equimolares Gemisch eines entsprechenden Aldehydes der Formel V

R₂―CH=O (V)

und einer Säure der Formel VI 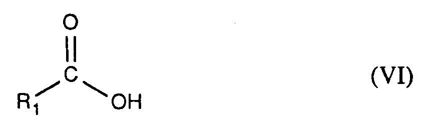
mit einem Überschuss eines Thionylhalogenides der Formel SO₂(R₃)₂ umsetzt. Beide Varianten dieses Verfahrens haben entschiedene Nachteile. Insbesondere ist bekannt, dass das gemäss der erstgenannten Variante erhaltenen Produkt durchwegs durch etwa 10 Mol-% des entsprechenden Bis(α-halogenalkyl)ethers verunreinigt ist. Gemäss der zweitgenannten Variante wird, wie das Vergleichbeispiel zeigt, sogar eine erheblich grössere Menge desselben gebildet. Bis(α-halogenalkyl)ether werfen jedoch auf Grund ihrer, insbesondere bei den niederen Homologen der Reihe sehr hohen, Toxizität erhebliche sicherheitstoxikologische Probleme auf. Dieses unerwünschte Nebenprodukt kann nur sehr schwierig abgetrennt und nur mit grossem Aufwand annähernd vollständig entfernt werden. Ausserdem werden neben Schwefeldioxid und Chlorwasserstoff stets eine Vielzahl weiterer Nebenprodukte gebildet, gemäss der erstgenannten Variante beispielsweise Acylalacetale der Formel VII 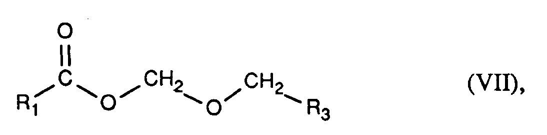
Anhydride der Formel VIII 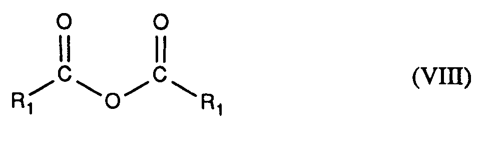
und Acylale der Formel II und gemäss der zweitgenannten Verfahrensvariante Säurechloride der Formel IV. Diese sowie überschüssiger Aldehyd der Formel V erschweren die Isolierung des gewünschten Produktes sehr stark.

Es hat deshalb nicht an Versuchen gefehlt, Verfahren zur Herstellung von Verbindungen der Formel I zu entwickeln, die die genannten Nachteile vermeiden. Die bislang bekannt gewordenen Lösungsvorschläge sind jedoch ebenfalls sicherheitstoxikologisch bedenklich oder zu aufwendig bzw. zu komplex, um industriell anwendbar zu sein.

Beispielsweise wurde vorgeschlagen, die Säure der Formel VI in Form eines Alkalimetallsalzes mit einem entsprechenden Chlorsulfonsäure(α-chlor)alkylester der Formel IX 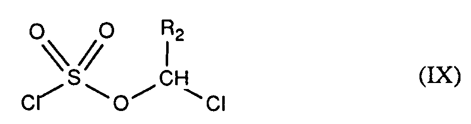
umzusetzen. Jedoch sind Reagentien der Formel IX wenig stabil und werfen auf Grund ihrer hohen Toxizität ihrerseits erhebliche sicherheitstoxikologische Probleme auf, was dem Einsatz dieses Verfahrens im technischen Masstab entgegenstehen.

Einem weiteren Vorschlag zufolge setzt man Methylendiacetat oder Methylendibenzoat (II; R₁= Methyl oder Phenyl, R₂= Wasserstoff) in Gegenwart der 0.05- bis 0.1-fachen molaren Menge eines Zinkhalogenides mit Trimethylbromsilan bzw. in Gegenwart der 0.2-fachen molaren Menge Aluminiumtrichlorid bei 120° C mit Trimethylchlorsilan um. Das Verfahren ist jedoch für die Herstellung von Verbindungen der Formel I, worin R₃ Chlor ist, nur sehr bedingt geeignet. So konnte bei der Umsetzung von Methylendibenzoat Chlormethylbenzoat nur in etwa 50 %-iger Ausbeute isoliert werden. Ausgehend vom Methylendiacetat enthielt das Reaktionsgemisch, wie durch Auswertung des ¹H-NMR-Spektrums ermittelt werden konnte, zwar etwa 40 Mol-% Chlormethylacetat; dieses konnte jedoch nicht isoliert werden. Als weiterer Nachteil kommt hinzu, dass stets in equimolarer Menge Gemische von Oligo- und Polysilanolen als unerwünschte, schwerflüchtige Nebenprodukte gebildet werden, die im industriellen Masstab nur sehr aufwendig entsorgt werden können.

Der Erfindung lag dementsprechend die bislang noch nicht gelöste Aufgabe zugrunde, ein Verfahren zur Herstellung von Verbindungen der Formel I zu entwickeln, welches die Nachteile der bekannten Verfahren vermeidet. Diese Aufgabe wird durch das erfindungsgemässe Verfahren sehr gut gelöst.

Das erfindungsgemässe Verfahren beruht auf der im Lichte des Standes der Technik überraschenden Feststellung, dass als Halogendonator anstelle hochtoxischer und/oder schwer handhabbarer Spezialragentien eine Halogenwasserstoffsäure der Formel III verwendet werden kann, ohne dass Ausbeute, Umsatzgeschwindigkeit und Produktreinheit beeinträchtigt werden, und dass so die Bildung von Bis(α-halogenalkyl)ethern sehr weitgehend vermieden werden kann. So ergibt sich aus dem Ausführungsbeispiel, dass erfindungsgemäss etwa 100-mal weniger Bis(chlormethyl)ether gebildet wird als bei der bereits optimierten bekannten Verfahrensweise gemäss dem Vergleichsbeispiel. Als weiterer Vorteil kommt hinzu, dass die Umsetzung sehr übersichtlich verläuft und weniger Nebenprodukte gebildet werden.

Die Umsetzung erfolgt üblicherweise unter Verwendung der mindestens equimolaren Menge einer Halogenwasserstoffsäure der Formel III (bezogen auf die molare Menge der Verbindung der Formel III), in An- oder Abwesenheit von Lösungs- oder Verdünnungsmitteln, vorteilhaft bei erhöhter Temperatur und mit anschliessender destillativer Aufarbeitung.

Die Umsetzung erfolgt vorzugsweise in Gegenwart einer Lewissäure als Katalysator unter Atmosphärendruck oder erhöhtem Druck, oder in Abwesenheit eines Lewiskatalysators bei Atmosphärendruck oder vorteilhaft bei erhöhtem Druck. Der Druck kann vorzugsweise bis zu 250 bar betragen, insbesondere bis zu 50 bar, z. B. bis zu 10 bar.

Stärker bevorzugt ist die Umsetzung in Gegenwart einer Lewissäure, die erforderlichenfalls unter geringem Überdruck erfolgt.

Als Lewissäuren werden erfindungsgemäss katalytische Mengen, beispielsweise die etwa 0.01- bis etwa 0.1-fache, wie etwa 0.02 bis etwa 0.05-fache molare Menge (bezogen auf die molare Menge der Verbindung der Formel II) eines Halogenides eines Metalles der Gruppen IIb, IIIb und IVb des Periodischen Systems der Elemente, wie entsprechende Zink-, Zinn- Zirkon- und Aluminiumhalogenide, insbesondere Zinkhalogenide der Formel Zn(R₃)₂ (X), verwendet.

Als Lösungsmittel kommem beispielsweise Halogenalkane oder Halogenaromaten, wie Di-, Tri- oder Tetrachlor-C₁-C₄-alkane, z.B. Methylenchlorid, Trichlorethan oder Chlorbenzol, in Betracht. Vorteilhaft kann die erfindungsgemässe Umsetzung jedoch ohne Lösungsmittel vorgenommen werden, wobei die Halogenwasserstoffsäure der Formel III in gasförmigem Aggregatzustand eingeleitet wird, erforderlichenfalls unter geringem Überdruck, beispielsweise unter einem Überdruck von etwa 10 bis 100 mbar, insbesondere von etwa 10 bis etwa 30 mbar.

Vorteilhaft wird ein geringer, beispielsweise etwa 1.05-facher bis etwa 1.75-facher, wie etwa 1.05-facher bis etwa 1.5-facher, insbesondere etwa 1.05-facher bis etwa 1.15-facher, molarer Überschuss an Halogenwasserstoffsäure verwendet.

Die Umsetzung wird vorteilhaft bei erhöhter Temperatur vorgenommen, vorzugsweise im Temperaturbereich von 0 °C bis 150 °C, beispielsweise von etwa 40° C bis etwa 120°C, z. B. von etwa 50 °C bis 100 °C, wie von etwa 50° C bis etwa 100° C, insbesondere von etwa 50 °C bis 80 °C, sehr bevorzugt bei etwa 60 °C.

Die destillative Abtrennung des Reaktionsproduktes erfolgt vorteilhaft unter vermindertem Druck, beispielsweise bei etwa 1 mbar bis etwa 50 mbar, insbesondere bei etwa 10 mbar bis etwa 30 mbar.

In einer bevorzugten Ausführungsform des erfindungsgemässen Verfahrens erwärmt man ein Gemisch eines Acylals der Formel II und der etwa 0.02- bis 0.05-fachen molaren Menge eines Zinkhalogenides der Formel IX auf etwa 50 °C bis 70° C, leitet über etwa 2 bis 10, insbesondere etwa 4 bis 6, Stunden einen etwa 1.05-fachen bis etwa 1.5-fachen, insbesondere etwa 1.05-fachen bis etwa 1.15-fachen, Überschuss an Halogenwasserstoff ein und destilliert unter vermindertem Druck, beispielsweise bei etwa 10 mbar bis etwa 30 mbar.

Die Ausgangsverbindungen der Formel II sind bekannt oder werden nach an sich bekannten Verfahren hergestellt.

Beispielsweise können die Acylale (Aldehydacylate) der Formel II hergestellt werden nach einem der in "Houben-Weyl - Methoden der organischen Chemie", E. Müller et al. (Herausgeber), Band 7, Teil 1,4. Auflage, Georg Thieme Verlag, Stuttgart, S. 442 genannten Verfahren, oder nach Kochhar et al., J. Org. Chem. 48, 1765(1983), Olah et al., Synthesis p. 962, (1982) oder Michie et al., Synthesis p. 824, (1981).

Die nachfolgen Beispiele dienen zur Illustration der Erfindung; Temperaturen sind in Celsiusgraden, Drucke in mbar angegeben.

Ausführungsbeispiel: 216 g Methylendipivalat (II; R₁= tert.-Butyl, R₂= Wasserstoff) werden auf etwa 60 ° erwärmt, mit 4.5 g Zinkchlorid versetzt Unter Rühren werden dann innerhalb von 5 Stunden insgesamt 38.5 g Chlorwasserstoff eingeleitet. In einem Aliquot wird die Zusammensetzung des Reaktionsgemisches bestimmt Die Reaktionsbilanz ist folgende:

| | |
|---|---|
| 143.0 g | Chlormethylpivalat |
| 4.5 g | Zinkchlorid |
| 96.9 g | Pivalinsäure |
| 0.4 g | Bis(chlormethyl)ether |
| 10.0 g | Methylendipivalat |

Das Reaktionsgemisch wird dann durch Zugabe von wenig Wasser hydrolysiert und unter vermindertem Druck (etwa 20 mbar) destillativ aufgetrennt; man erhält mindestens 99 %-ig reines Chlormethylpivalat` welches weniger als 0,1 ppm des toxischen Bis(chlormethyl)ethers enthält, in einer Ausbeute von 95 % der Theorie.

### Vergleichsbeispiel:

1.7 g Zinkchlorid werden mit 0,8 ml Wasser verrührt und unter Rühren vorsichtig mit 180.0 g Thionylchlorid versetzt. Man lässt 1 Stunde bei Raumtemperatur nachrühren und fügt dann unter Rühren bei 70° portionsweise eine Suspension von 42.1 g Paraformaldehyd in 85.0g geschmolzener Pivalinsäure hinzu. Man erwärmt 1 Stunde unter Rühren auf 100°. Im Verlaufe der Reaktion werden insgesamt 36.5 g Chlorwasserstoff und 80 g Schwefeldioxid freigesetzt. In einem Aliquot wird die Zusammensetzung des Reaktionsgemisches bestimmt. Die Reaktionsbilanz ist unter Berücksichtigung der separat aufgefangenen flüchtigen Bestandteile folgende:

| | |
|---|---|
| 106.5 g | Chlormethylpivalat |
| 80.0 g | Schwefeldioxid |
| 13.0 g | Pivalinsäurechlorid |
| 30.0 g | Thionylchlorid |
| 52.0 g | Bis(chlormethyl)ether |
| 36.5 g | Chlorwasserstoff |
| 1.7 g | Zinkchlorid |

Das Reaktionsgemisch wird unter vermindertem Druck (etwa 20 mbar) destillativ aufgetrennt; man erhält etwa 99 %-ig reines Chlormethylpivalat, Ausbeute 85.2 % der Theorie.

## Patentansprüche

1. Verfahren zur Herstellung von halogenierten Carbonsäureestern der Formel I 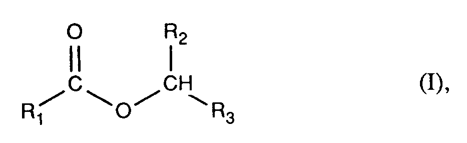 worin R₁ Alkyl, Alkenyl, Alkinyl, Cycloalkyl, Aralkyl oder Aryl bedeutet, R₂ für Wasserstoff, Alkyl oder Cycloalkyl steht und R₃ Halogen der Ordnungszahl 9 bis und mit 53 darstellt, dadurch gekennzeichnet, dass man ein Acylal der Formel II 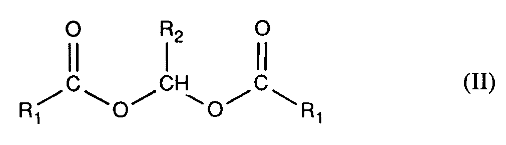 mit einem Halogenwasserstoff der Formel
H-R₃ (III)
umsetzt.

2. Verfahren gemäss Anspruch 1, in dem man die Reaktion in Gegenwart einer Lewissäure durchführt.

3. Verfahren gemäss einem der Ansprüche 1 oder 2, in dem man das Acylal der Formel II mit der mindestens equimolaren Menge eines Halogenwasserstoffes der Formel III umsetzt.

4. Verfahren gemäss einem der Ansprüche 1 oder 2, in dem man, bezogen auf die molare Menge des Acylals der Formel II, einen 1.05-fachen bis 1.75-fachen Überschuss an Halogenwasserstoffsäure der Formel III verwendet.

5. Verfahren gemäss einem der Ansprüche 1 bis 4, in dem man im Temperaturbereich von 0 °C bis 150 °C arbeitet.

6. Verfahren gemäss einem der Ansprüche 2 bis 5, in dem man, bezogen auf die molare Menge des Acylals der Formel II, als Lewissäure eine katalytische 0.01 bis 0.1-fache molare Menge eines Halogenides eines Metalles der Gruppen IIb, IIIb und IVb des Periodischen Systems der Elemente verwendet.

7. Verfahren gemäss einem der Ansprüche 1 bis 6, in dem man die destillative Abtrennung des Reaktionsproduktes unter vermindertem Druck von 1 mbar bis 50 mbar vornimmt.

8. Verfahren gemäss einem der Ansprüche 1 bis 7, in dem man die Ausgangsverbindungen so wählt, dass man Verbindungen der Formel I, worin R₁ C₁-C₁₄-Alkyl, C₂-C₇-Alkenyl, C₃-C₇-Alkinyl, 3- bis 8-gliedriges Cycloalkyl, unsubstituiertes oder durch C₁-C₄-Alkyl, C₁-C₄-Alkoxy, Halogen, Trifluormethyl und/oder Nitro substituiertes Phenyl oder Naphthyl oder unsubstituiertes oder im Phenyl durch C₁-C₄-Alkyl, C₁-C₄-Alkoxy, Halogen, Trifluormethyl und/oder Nitro substituiertes Mono-, Di- oder Triphenyl-C₁-C₄-alkyl bedeutet, R₂ für Wasserstoff, C₁-C₇-Alkyl oder 3- bis 8-gliedriges Cycloalkyl steht und R₃ Halogen der Ordnungszahl 9 bis und mit 53 darstellt bedeutet, herstellt.

9. Verfahren gemäss einem der Ansprüche 1 bis 7, in dem man die Ausgangsverbindungen so wählt, dass man Verbindungen der Formel I, worin R₁ C₁-C₇-Alkyl bedeutet, R₂ für Wasserstoff oder C₁-C₄-Alkyl steht und R₃ Chlor oder Brom darstellt, herstellt.

10. Verfahren gemäss einem der Ansprüche 1 bis 7, in dem man die Augangsverbindungen so wählt, dass man Verbindungen der Formel I, worin R₁ Methyl, Ethyl oder sekundär-lineares oder verzweigtes C₃-C₇-Alkyl bedeutet, R₂ für Wasserstoff oder lineares C₁-C₄-Alkyl steht und R₃ Chlor darstellt, herstellt.

11. Verfahren gemäss einem der Ansprüche 1 bis 7, in dem man die Ausgangsverbindungen so wählt, dass man Verbindungen der Formel 1, worin R₁ Methyl, Ethyl oder sekundär-lineares oder verzweigtes C₃-C₇-Alkyl bedeutet R₂ für Wasserstoff steht und R₃ Chlor darstellt, herstellt.

12. Verfahren gemäss einem der Ansprüche 1 bis 7, in dem man die Ausgangsverbindungen so wählt, dass man Chlormethylpivalat (I; R₁ = tert.-Butyl, R₂ = Wasserstoff, R₃= Chlor) herstellt.

13. Verfahren gemäss einem der Ansprüche 1 bis 7, worin man Chlormethylpivalat (der Formel I; R₁ = tert.-Butyl, R₂ = Wasserstoff) herstellt, indem man Methylendipivalat (der Formel II; R₁ = tert.-Butyl, R₂ = Wasserstoff) auf etwa 60 °C erwärmt, mit 0,033 mol Zinkchlorid pro mol Methylendipivalat versetzt, innerhalb von 5 Stunden 1,057 mol Chlorwasserstoff je mol Chlormethylpivalat einleitet, das Reaktionsgemisch dann durch Zugabe von wenig Wasser hydrolysiert und bei etwa 20 mbar destillativ auftrennt.

## Claims

1. A process for the preparation of a halogenated carboxylic acid ester of formula I 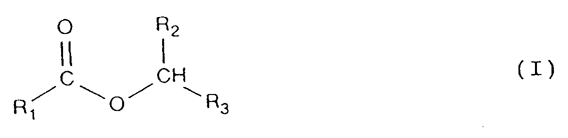 wherein R₁ is alkyl, alkenyl, alkynyl, cycloalkyl, aralkyl or aryl, R₂ is hydrogen, alkyl or cycloalkyl, and R₃ is halogen having an atomic number of from 9 up to and including 53, which process comprises reacting an acylal of formula II 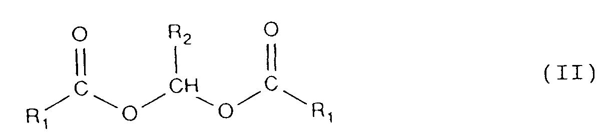 with a hydrogen halide of the formula
H-R₃ (III).

2. A process according to claim 1 wherein the reaction is carried out in the presence of a Lewis acid.

3. A process according to either claim 1 or claim 2 wherein the acylal of formula II is reacted with at least an equimolar amount of a hydrogen halide of formula III.

4. A process according to either claim 1 or claim 2 wherein, referring to the molar amount of the acylal of formula II, a 1.05- to 1.75-fold excess of the hydrogen halide of formula III is used.

5. A process according to any one of claims 1 to 4 wherein the reaction is carried out in a temperature range of from 0°C to 150°C.

6. A process according to any one of claims 2 to 5 wherein, referring to the molar amount of the acylal of formula II, there is used a catalytic 0.01- to 0.1-fold molar amount of a halide of a metal of groups IIb, IIIb and IVb of the periodic system of the elements as Lewis acid.

7. A process according to any one of claims 1 to 6 wherein the separation of the reaction product by distillation is carried out under reduced pressure of from 1 mbar to 50 mbar.

8. A process according to any one of claims 1 to 7 wherein the starting materials are so selected that there is prepared a compound of formula I wherein R₁ is C₁-C₁₄alkyl, C₂-C₇alkenyl, C₃-C₇alkynyl or 3- to 8-membered cycloalkyl; or is phenyl or naphthyl each of which is unsubstituted or substituted by C₁-C₄alkyl, C₁-C₄alkoxy, halogen, trifluoromethyl and/or by nitro, or mono-, di- or tri-phenyl-C₁-C₄alkyl that is unsubstituted or substituted in the phenyl moiety by C₁-C₄alkyl, C₁-C₄alkoxy, halogen, trifluoromethyl and/or by nitro, R₂ is hydrogen, C₁-C₇alkyl or 3- to 8-membered cycloalkyl, and R₃ is halogen having an atomic number of from 9 up to and including 53.

9. A process according to any one of claims 1 to 7 wherein the starting materials are so selected that there is prepared a compound of formula I wherein R₁ is C₁-C₇-alkyl, R₂ is hydrogen or C₁-C₄alkyl, and R₃ is chlorine or bromine.

10. A process according to any one of claims 1 to 7 wherein the starting materials are so selected that there is prepared a compound of formula I wherein R₁ is methyl, ethyl or secondary-linear or branched C₃-C₇alkyl, R₂ is hydrogen or linear C₁-C₄alkyl, and R₃ is chlorine.

11. A process according to any one of claims 1 to 7 wherein the starting materials are so selected that there is prepared a compound of formula I wherein R₁ is methyl, ethyl or secondary-linear or branched C₃-C₇alkyl, R₂ is hydrogen and R₃ is chlorine.

12. A process according to any one of claims 1 to 7 wherein the starting materials are so selected that chloromethyl pivalate (I; R₁ = tert-butyl, R₂ = hydrogen, R₃ = chlorine) is prepared.

13. A process according to any one of claims 1 to 7 wherein chloromethyl pivalate (of formula I; R₁ = tert-butyl, R₂ = hydrogen) is prepared by heating methylene dipivalate (of formula II; R₁ = tert-butyl, R₂ = hydrogen) to approximately 60°C, adding 0.033 mol of zinc chloride per mole of methylene dipivalate, introducing within a period of 5 hours 1.057 mol of hydrogen chloride per mole of chloromethyl pivalate, then hydrolysing the reaction mixture by the addition of a small amount of water, and separating the reaction mixture by distillation at approximately 20 mbar.

## Revendications

1. Procédé de préparation d'esters halogénés d'acides carboxyliques de formule I 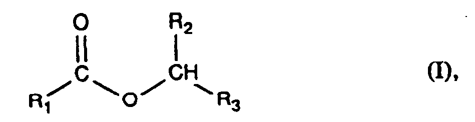 dans laquelle R₁ représente un groupe alkyle, alcényle, alcynyle, cycloalkyle, aralkyle ou aryle, R₂ représente l'hydrogène, un groupe alkyle ou cycloalkyle et R₃ un halogène de numéro atomique 9 à 53 inclus, caractérisé en ce que l'on fait réagir un acylal de formule II 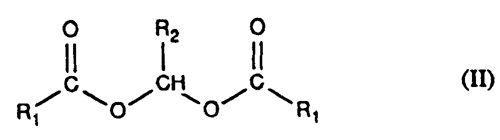 avec un halogénure d'hydrogène de formule
H-R₃

2. Procédé selon revendication 1, dans lequel on effectue la réaction en présence d'un acide de Lewis.

3. Procédé selon l'une des revendications 1 ou 2, dans lequel on fait réagir l'acylal de formule II avec la quantité au moins équimoléculaire d'un halogénure d'hydrogène de formule III.

4. Procédé selon l'une des revendications 1 ou 2, dans lequel, par rapport à la quantité molaire de l'acylal de formule II, on utilise l'halogénure d'hydrogène de formule III en excès de 1,05 à 1,75 fois.

5. Procédé selon l'une des revendications 1 à 4,dans lequel on opère dans l'intervalle de température de 0 à 150° C.

6. Procédé selon l'une des revendications 2 à 5, dans lequel, par rapport à la quantité molaire de l'acylal de formule II, on utilise un acide de Lewis consistant en un halogénure d'un métal des groupes IIb, IIIb et IVb de la Classification Périodique des Eléments en quantité catalytique de 0,01 à 0,1 fois molaire.

7. Procédé selon l'une des revendications 1 à 6, dans lequel on procède à la séparation par distillation du produit de réaction sous un vide de 1 à 50 mbar.

8. Procédé selon l'une des revendications 1 à 7, dans lequel on choisit les composés de départ de manière à préparer des composés de formule I dans laquelle R₁ représente un groupe alkyle en C₁-C₁₄, alcényle en C₂-C₇, alcynyle en C₃-C₇, cycloalkyle de 3 à 8 chaînons, phényle ou naphtyle non substitué ou substitué par des groupes alkyle en C₁-C₄, alcoxy en C₁-C₄, des halogènes, des groupes trifluorométhyle et/ou nitro, ou mono-, di- ou tri-phénylalkyle en C₁-C₄ non substitué ou substitué dans la partie phényle par des groupes alkyle en C₁-C₄, alcoxy en C₁-C₄, dès halogènes, des groupes trifluorométhyle et/ou nitro, R₂ représente l'hydrogène, un groupe alkyle en C₁-C₇ ou cycloalkyle de 3 à 8 chaînons et R₃ un halogène de numéro atomique 9 à 53 inclus.

9. Procédé selon l'une des revendications 1 à 7, dans lequel on choisit les composés de départ de manière à préparer des composés de formule I dans laquelle R₁ représente un groupe alkyle en C₁-C₇, R₂ l'hydrogène ou un groupe alkyle en C₁-C₄ et R₃ le chlore ou le brome.

10. Procédé selon l'une des revendications 1 à 7, dans lequel on choisit les composés de départ de manière à préparer des composés de formule I dans laquelle R₁ représente un groupe méthyle, éthyle ou un groupe alkyle secondaire à chaîne droite ou ramifiée en C₃-C₇, R₂ représente l'hydrogène ou un groupe alkyle à chaîne droite en C₁-C₄ et R₃ représente le chlore.

11. Procédé selon l'une des revendications 1 à 7, dans lequel on choisit les composés de départ de manière à préparer des composés de formule I dans laquelle R₁ représente un groupe méthyle, éthyle ou un groupe alkyle secondaire à chaîne droite ou ramifiée, R₂ représente l'hydrogène et R₃ le chlore.

12. Procédé selon l'une des revendications 1 à 7 dans lequel on choisit les composés de départ de manière à préparer le pivalate de chlorométhyle (I ; R₁ = tert-butyle, R₂ = hydrogène, R₃ = chlore).

13. Procédé selon l'une des revendications 1 à 7, dans lequel on prépare le pivalate de chlorométhyle (de formule I ; R₁ = tert-butyle, R₂ = hydrogène) en chauffant le dipivalate de méthylène (de formule II ; R₁ = tert-butyle, R₂ = hydrogène) à une température d'environ 60° C, en ajoutant 0,033 mol de chlorure de zinc par mol de dipivalate de méthylène, en injectant en 5 h 1,057 mol de chlorure d'hydrogène par mol de pivalate de chlorométhyle, en hydrolysant ensuite le mélange de réaction par addition d'un peu d'eau et en fractionnant par distillation sous un vide d'environ 20 mbar.
